(19) Europäisches Patentamt
European Patent Office
Office européen des brevets

(11) EP 4 332 157 A1

(12) EUROPEAN PATENT APPLICATION
published in accordance with Art. 153(4) EPC

(43) Date of publication:
**06.03.2024 Bulletin 2024/10**

(21) Application number: **22936639.8**

(22) Date of filing: **21.09.2022**

(51) International Patent Classification (IPC):
***C08J 11/10*** (2006.01) ***C08J 11/14*** (2006.01)
***C08J 11/22*** (2006.01)

(52) Cooperative Patent Classification (CPC):
**C08J 11/10; C08J 11/14; C08J 11/22; C08J 11/24;**
Y02W 30/62

(86) International application number:
**PCT/KR2022/014120**

(87) International publication number:
**WO 2023/195586 (12.10.2023 Gazette 2023/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.04.2022 JP 2022062848**

(71) Applicant: **LG Chem, Ltd.**
**Seoul 07336 (KR)**

(72) Inventors:
- **SHINNOSUKE, Koji**
  **Yokohama, Kanagawa 220--0011 (JP)**
- **HIROKI, Ishizaki**
  **Yokohama, Kanagawa 220--0011 (JP)**

(74) Representative: **Hoffmann Eitle**
**Patent- und Rechtsanwälte PartmbB**
**Arabellastraße 30**
**81925 München (DE)**

(54) **TREATMENT APPARATUS, METHOD FOR PREPARING DEGRADATION PRODUCT, AND TREATMENT METHOD**

(57) Provided are a processing apparatus, a method of manufacturing a decomposition product, and a processing method, which are capable of improving efficiency in decomposing and processing a polymer compound. The processing apparatus according to the present disclosure is a processing apparatus for a polymer compound, the processing apparatus including a container configured to accommodate a polymer compound and a liquid medium, and a plasma generator disposed in the container and configured to perform plasma processing on the liquid medium.

[Figure 1]

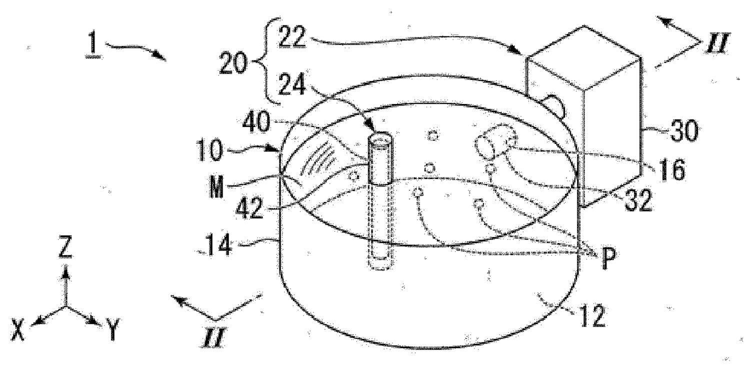




Processed by Luminess, 75001 PARIS (FR)

## Description

[Technical Field]

This application claims priority to and the benefit of Japanese Patent Application No. 2022-62848 filed with the Japanese Patent Office on April 5, 2022, the entire contents of which are incorporated herein by reference.
Embodiments of the present invention relate to a processing apparatus, a method of manufacturing a decomposition product, and a processing method.

[Background Art]

A chemical recycling technology, which performs a depolymerization reaction to decompose a polymer compound into monomers and use the monomers that are raw materials, has attracted attention and has been developed. Patent Document 1 discloses a technology that decomposes a polymer compound by using a catalyst. Patent Document 2 discloses a technology that performs a depolymerization reaction under a high temperature and a high pressure by irradiating polyester with microwaves in the presence of water.

[Documents of Related Art]

[Patent Documents]

Patent Document 1: Japanese Patent Laid-Open No. 2008-88096
Patent Document 2: Japanese Patent Laid-Open No. 2015-168741

[Detailed Description of the Invention]

[Technical Problem]

However, in the case of the technology of Patent Document 1, it is necessary to eliminate the catalyst during a post-process. The technology of Patent Document 2 requires a special reaction condition such as a high temperature and a high pressure. Therefore, there is a need to more efficiently perform the decomposition of the polymer compound.
The present invention has been made in an effort to provide a processing apparatus, a method of manufacturing a decomposition product, and a processing method, which are capable of improving efficiency in decomposing and processing a polymer compound.

[Technical Solution]

The present invention may include the following aspects.

[1] A processing apparatus for a polymer compound, the processing apparatus including: a container configured to accommodate a polymer compound and a liquid medium; and a plasma generator disposed in the container and configured to perform plasma processing on the liquid medium.
[2] In [1], the plasma generator includes: an electromagnetic wave transmission part configured to transmit electromagnetic waves; and an electromagnetic wave receiving part configured to receive the electromagnetic waves from the electromagnetic wave transmission part.
[3] In [2], the electromagnetic wave receiving part performs the plasma processing on the liquid medium in response to the reception of the electromagnetic waves.
[4] In [2], the electromagnetic wave is a microwave with a frequency of 100 MHz or more and 300 GHz or less.
[5] In [2], the electromagnetic wave receiving part includes a metallic material.
[6] In [2], the electromagnetic wave receiving part includes: a core having conductivity; and a dielectric material sheath configured to at least partially cover the core.
[7] In [2], the electromagnetic wave receiving part is disposed inside the container and comes into contact with the liquid medium.
[8] In [2], at least a part of the electromagnetic wave transmission part is disposed inside the container.
[9] In [2], the electromagnetic wave transmission part is disposed outside the container, and a portion of the container, through which the electromagnetic waves from the electromagnetic wave transmission part at least pass, is made

of a nonmetallic material.

[10] A method, which manufactures a decomposition product by processing a polymer compound, the method including: bringing the polymer compound into contact with plasma generated from a liquid medium.

[11] In [10], the method further includes: emitting electromagnetic waves to the electromagnetic wave receiving part in a state in which the liquid medium is in contact with the electromagnetic wave receiving part.

[12] In [11], the electromagnetic wave receiving part performs the plasma processing on the liquid medium in response to the reception of the electromagnetic waves.

[13] In any one of [10], [11], and [12], the method further includes: generating a radical of the liquid medium by performing plasma processing on the liquid medium.

[14] In [10], the method further includes: performing plasma processing on the liquid medium in a state in which the polymer compound is dissolved or dispersed in the liquid medium that is not converted into plasma.

[15] In [10], the polymer compound comes into contact with the plasma generated from the liquid medium under a condition of a temperature of 10°C or more and 150°C or less.

[16] In [10], the polymer compound configured to come into contact with the plasma generated from the liquid medium under a condition of a pressure of 0.05 MPa or more and less than 1.5 MPa.

[17] In [10], the liquid medium includes polar molecules.

[18] In [10], the polymer compound comes into contact with the plasma generated from the liquid medium in a state in which no catalyst for a decomposition reaction for a polymer compound is present.

[19] In [10], a decomposition product decomposed from the polymer compound is produced as the polymer compound comes into contact with the plasma generated from the liquid medium.

[20] A method, which processes a polymer compound, the method including: bringing the polymer compound into contact with plasma generated from a liquid medium.

[Advantageous Effects]

According to the present invention, it is possible to provide a processing apparatus, a method of manufacturing a decomposition product, and a processing method, which are capable of improving efficiency in decomposing and processing a polymer compound.

[Brief Description of Drawings]

FIG. 1 is a perspective view illustrating a processing apparatus according to a first embodiment.

FIG. 2 is a cross-sectional view taken along line II-II in FIG. 1 and illustrating the processing apparatus according to the first embodiment.

FIG. 3 is a cross-sectional view illustrating a processing apparatus according to a second embodiment.

[Explanation of Reference Numerals and Symbols]

1...Processing apparatus, 10...Container, 12...Bottom wall, 14...Sidewall, 16...Mounting part, 20...Plasma generator, 22...Electromagnetic wave transmission part, 30...Electromagnetic wave output part, 32...Electromagnetic wave emission part, 24...Electromagnetic wave receiving part, 40...Core, 42...Dielectric sheath, P...Polymer compound, M...Liquid medium

[Best Mode]

Hereinafter, a processing apparatus, a method of manufacturing a decomposition product, and a processing method according to embodiments will be described with reference to the drawings. In addition, the drawings are schematic or conceptual. A relationship between a thickness and a width of each part, a ratio between sizes of parts, and the like are not necessarily the same as those of actual parts. In addition, even though the identical parts are indicated, dimensions or ratios of the identical parts are sometimes differently illustrated in accordance with the drawings. In addition, an XYZ coordinate illustrated in the drawings is defined for the convenience of description but does not limit the invention.

<First Embodiment>

A processing apparatus 1 according to a first embodiment and a processing method using the processing

apparatus 1 will be described with reference to FIGS. 1 and 2.

FIG. 1 is a perspective view illustrating the processing apparatus 1 according to the first embodiment.

FIG. 2 is a cross-sectional view taken along line II-II in FIG. 1 and illustrating the processing apparatus 1 according to the first embodiment.

[Processing Apparatus 1]

According to the present embodiment, the processing apparatus 1 is provided as a processing apparatus 1 for a polymer compound P and includes a container 10 configured to accommodate the polymer compound P and a liquid medium M, and a plasma generator 20 disposed in the container 10 and configured to perform plasma processing on the liquid medium M.

The processing apparatus 1 refers to an apparatus for processing the polymer compound P. Specifically, the processing apparatus 1 may decompose or depolymerize the polymer compound P.

(Container 10)

The container 10 provides a reaction field for a processing reaction of the polymer compound P. As illustrated in FIGS. 1 and 2, the container 10 has a bottom wall 12, and a sidewall 14 protruding upward from the bottom wall 12. The container 10 may further include a top wall installed at an upper side of the sidewall 14. The container 10 has an internal space defined by the bottom wall 12 and the sidewall 14, and optionally, by the top wall. The internal space of the container 10 may be opened to the outside or closed.

A shape of the container 10 may be a cylindrical shape having an internal space, a polyprismatic shape, or a container shape having a bottom. However, the shape of the container 10 is not limited to the above-mentioned example, and the container 10 may have any shape as long as the container 10 may accommodate the polymer compound P and the liquid medium M.

A material of the container 10 may be a conductor, a semiconductor, or an insulator without being particularly limited. Examples of the material of the container 10 may include metal, glass, ceramics, enamel, rubber, and plastic. The container 10 made of an inorganic material is advantageous in sufficiently preventing an influence of a decomposition reaction.

A mounting part 16 on which an electromagnetic wave transmission part 22 to be described below is mounted is installed on the sidewall 14. For example, as illustrated in FIGS. 1 and 2, the mounting part 16 is formed as a hole into which the electromagnetic wave transmission part 22 is inserted.

(Liquid Medium M)

The liquid medium M mediates the processing on the polymer compound P by the processing apparatus 1. The liquid medium M may serve as a dispersion medium or solvent for the polymer compound P. In the present specification, the "liquid medium" means a medium that is a liquid under atmospheric pressure. For example, the liquid medium M is a liquid at a temperature of 20°C and under atmospheric pressure. For example, a boiling point of the liquid medium M may be, but not particularly limited to, 20°C or less, 10°C or less, or 0°C or less. For example, the boiling point of the liquid medium M may be, but not particularly limited to, 20°C or more, 30°C or more, 40°C or more, 50°C or more, 60°C or more, 70°C or more, 80°C or more, 90°C or more, 100°C or more, 150°C or more, or 200°C or more. Properties, such as a viscosity, of the liquid medium M are not particularly limited. Any additive such as a dispersant or a viscosity controlling agent may added to the liquid medium M.

The type of liquid medium M is not particularly limited, and one or more types of liquid material may be used. Examples of the liquid medium M may include water, alcohol, ketone, ether, ester, carboxylic acid, amine, aromatic compound, nitrile, and alkyl halide. Examples of alcohol may include methanol, ethanol, propanol, butanol, ethylene glycol, diethylene glycol, glycerol, and allyl alcohol. Examples of ketone may include acetone, acetyl acetone, and ethyl methyl ketone. Examples of ether may include dimethyl ether, ethyl methyl ether, diethyl ether, and dibutyl ether. Examples of ester may include ethyl acetate and butyl acetate. Examples of carboxylic acid may include formic acid, acetic acid, propionic acid, and benzoic acid. Examples of amine may include aniline, ethanol amine, and diethanol amine. Examples of aromatic compound may include benzene. Examples of nitrile may include acetonitrile. Examples of alkyl halide may include chloroform and dichloro methane. The liquid medium M may be a solution in which any material is dissolved or a dispersion liquid in which any material is dispersed.

For example, the liquid medium M contains polar molecules. This is advantageous because the liquid medium M has the polarity, and thus an interaction with electromagnetic waves, particularly, microwaves increases, such that plasma is easily produced. Particularly, the liquid medium M contains one or more materials selected from a group consisting of water, alcohol, ketone, ether, and ester.

(Polymer Compound P)

The polymer compound P is an object to be processed by the processing apparatus 1. The polymer compound P may be a polymer or oligomer made by polymerizing one or two or more types of monomers. For example, the polymer compound P may be, but not particularly limited to, polyolefin (polyethylene, polypropylene, etc.), polyester (polyethylene terephthalate, etc.), polyether, polyurethane, polyamide (polyacrylic amide etc.), polyimide, polycarbonate, polyacetal, epoxy resin, polystyrene, polyvinyl halide (polyvinyl chloride etc.), fluorine resin, polyvinyl alcohol, acrylonitrile·butadiene·styrene copolymer synthetic resin (ABS resin), synthetic rubber, and fiber reinforced plastic.

The polymer compound P is in contact with the liquid medium M in the container 10. Specifically, as illustrated in FIGS. 1 and 2, the polymer compound P is a particulate and may be dispersed in the liquid medium M or dissolved in the liquid medium M. However, the presence form of the polymer compound P is not limited to the above-mentioned example. For example, the polymer compound P is a lump sized to be visible and may be present in the liquid medium M. For example, the polymer compound P may be mounted on a bottom wall 12 or a mounting table installed in the container 10.

For example, a mass ratio between the polymer compound P and the liquid medium M is 0.001:99.999 to 50:50, 0.01:99.99 to 30:70, 0.1:99.9 to 20:80, 1:99 to 10:90, or 2:98 to 5:95.

(PLASMA GENERATOR 20)

The plasma generator 20 performs the plasma processing on the liquid medium M. Specifically, the plasma generator 20 may generate plasma from the liquid medium M by performing the plasma processing on the liquid medium M in the container 10. Specifically, as illustrated in FIGS. 1 and 2, the plasma generator 20 includes the electromagnetic wave transmission part 22 configured to transmit electromagnetic waves, and an electromagnetic wave receiving part 24 configured to receive electromagnetic waves from the electromagnetic wave transmission part 22.

The electromagnetic wave transmission part 22 transmits the electromagnetic waves to the electromagnetic wave receiving part 24. The electromagnetic wave transmission part 22 outputs the electromagnetic waves to generate plasma from the liquid medium M in cooperation with the electromagnetic wave receiving part 24. The configuration of the electromagnetic wave transmission part 22 is not particularly limited, and the electromagnetic wave transmission part 22 may have any configuration capable of transmitting electromagnetic waves. For example, as illustrated in FIGS. 1 and 2, the electromagnetic wave transmission part 22 includes an electromagnetic wave output part 30 configured to output electromagnetic waves, and an electromagnetic wave emission part 32 configured to emit the electromagnetic waves, which are outputted from the electromagnetic wave output part 30, to the electromagnetic wave receiving part 24. The electromagnetic wave output part 30 includes a power source, an oscillator configured to emit electromagnetic waves, and an amplifier configured to amplify the emitted electromagnetic waves. The electromagnetic wave emission part 32 serves as a transmission antenna for emitting electromagnetic waves into the container 10. The electromagnetic wave emission part 32 is mounted on the mounting part 16. Therefore, the electromagnetic wave emission part 32 is partially positioned inside the container 10 and partially positioned outside the container 10. Therefore, a part of the electromagnetic wave transmission part 22 is positioned inside the container 10, and the other part of the electromagnetic wave transmission part 22 is positioned outside the container 10. In addition, the entire electromagnetic wave transmission part 22 may be disposed in the container 10. That is, at least a part of the electromagnetic wave transmission part 22 may be disposed in the container 10. The electromagnetic wave emission part 32 may be integrated with the container 10, or the entire electromagnetic wave transmission part 22 may be integrated with the container 10.

The electromagnetic wave receiving part 24 is irradiated with the electromagnetic waves emitted from the electromagnetic wave transmission part 22, thereby allowing the electromagnetic wave receiving part 24 to emit electrons. For example, the electromagnetic wave is the microwave with a frequency of 100 MHz or more and 300 GHz or less. More specifically, the frequency of the electromagnetic wave may be 2.45 GHz, which is recognized for domestic use in Japan, or 915 MHz used for food thawing.

The electromagnetic wave receiving part 24 serves as a receiving antenna configured to receive the electromagnetic waves transmitted from the electromagnetic wave transmission part 22. The electromagnetic wave receiving part 24 emits electrons from a surface thereof in response to the reception of the electromagnetic waves from the electromagnetic wave transmission part 22. The emitted electrons attack and activate the liquid medium M in the container 10, thereby generate plasma from the liquid medium M. For example, the electromagnetic wave receiving part 24 includes a metallic material.

The electromagnetic wave receiving part 24 is disposed in the container 10. The electromagnetic wave receiving part 24 is in contact with the liquid medium M. Specifically, as illustrated in FIGS. 1 and 2, the electromagnetic wave receiving part 24 extends upward (in a Z direction) from the bottom wall 12 of the container 10 and has a long, thin shape. In addition, the electromagnetic wave receiving part 24 may be integrated with the container 10. The number of electromagnetic wave receiving parts 24 is not particularly limited.

The electromagnetic wave receiving part 24 includes a core 40 having conductivity, and a dielectric sheath 42 configured to at least partially cover the core 40. As illustrated in FIGS. 1 and 2, an outer surface of the core 40 is covered by the dielectric sheath 42.

The core 40 is a conductive member including a metallic material. When the electromagnetic waves (e.g., microwaves) are emitted, the core 40 emits free electrons from a surface thereof. The emitted electrons pass through the dielectric sheath 42 that covers the core 40, and the electrons are emitted from the electromagnetic wave receiving part 24 and generate plasma from the liquid medium M.

For example, a material of the core 40 is metal. Examples of the metallic material may include single metal such as copper, iron, aluminum, magnesium, titanium, vanadium, chromium, manganese, cobalt, nickel, zinc, germanium, palladium, indium, tin, silver, molybdenum, tantalum, tungsten, gold, and platinum, an alloy containing one or more of the above-mentioned elements as a main component, metal oxide having physical properties of metal, metal carbide, metal silicide, metal nitride, and a composite material containing the above-mentioned elements as a base.

The dielectric sheath 42 covers a part or the entirety of a periphery of the core 40. The dielectric sheath 42 inhibits elution of the core 40 to the liquid medium M. The dielectric sheath 42 inhibits a reaction between the metal element, which constitutes the core 40, and the liquid medium M or oxygen in air. Therefore, the dielectric sheath 42 may serve as a protective layer that prevents corrosion of the core 40. Meanwhile, the dielectric sheath 42 may at least partially transmit the electromagnetic waves emitted from the electromagnetic wave transmission part 22 and the electrons emitted from the core 40. In addition, when the electrons, which do not pass through the dielectric sheath 42 among the electrons emitted from the core 40, are accumulated inside the dielectric sheath 42, a potential difference may occur at two opposite sides of the dielectric sheath 42. Therefore, the dielectric sheath 42 generates dielectric polarization and generates charges on the surface of the dielectric sheath 42. All the electrons passing through the dielectric sheath 42 and the charges generated on the surface of the dielectric sheath 42 by the dielectric polarization may contribute to the generation of plasma from the liquid medium M.

A material of the dielectric sheath 42 is a dielectric material. Examples of the material of the dielectric sheath 42 may include glass and ceramics. Examples of glass may include amorphous silicon oxide (e.g., silicon dioxide $SiO_2$), soda lime glass, quartz glass, silicate glass, and borosilicate glass. Examples of ceramics may include an inorganic material (e.g., diamond, silicon, carbon fiber, silicon carbide, fullerene, or boron carbide) that does not include metal oxide, metal carbide, metal silicide, metal nitride, or metal element.

For example, a thickness of the dielectric sheath 42 is 1 um to 500 um, 10 um to 300 μm, or 50 um to 200 um. When the thickness is 1 μm or more, it is possible to ensure predetermined insulation and reduce a likelihood of an insulation breakdown. When the thickness is 500 μm or less, it is possible to reduce a likelihood that the electrons cannot pass through the dielectric sheath 42.

The dielectric sheath 42 is interposed between the core 40 and the external structure (e.g., the container 10) and serves to prevent the core 40 from being grounded. For example, when the core 40 is grounded by being in direct contact with the container 10 made of metal, the electrons of the core 40 excited by the electromagnetic waves come out to the container 10, and for this reason, there is a likelihood that the generation of plasma from the liquid medium M cannot be efficiently performed. That is, the dielectric sheath 42 electrically insulates the core 40 and the external structure. A portion of the dielectric sheath 42, which is positioned between the core 40 and the external structure, may have a thickness that does not substantially transmit the electrons. For example, as illustrated in FIG. 2, a bottom portion of the dielectric sheath 42, which is positioned between the core 40 and the bottom wall 12 may have a larger thickness than the other portions.

For example, a distance d between the core 40 and a tip of the electromagnetic wave emission part 32 illustrated in FIG. 2 satisfies Relationship Formula 1 below. For example, d=λ/4. For example, in case that the electromagnetic wave emission part 32 emits microwaves with a frequency of 2.45 GHz, d may be 27 mm or more and 34 mm or less. The configuration in which d satisfies Relationship Formula 1 is advantageous because the electromagnetic wave receiving part 24 stably generates plasma.

[Relationship Formula 1]

$$0.9\times\lambda/4 \leq d \leq 1.1\times\lambda/4 \quad (\lambda\text{: wavelength of electromagnetic wave})$$

[Method of Using Processing Apparatus 1]

Next, a method of using the processing apparatus 1 will be described.

According to the present embodiment, a method of processing the polymer compound P includes bringing the

polymer compound P into contact with the plasma generated from the liquid medium M.

As another embodiment, a method of manufacturing a decomposition product according to the present embodiment is a method of manufacturing a decomposition product by processing the polymer compound P, the method including bringing the polymer compound P into contact with the plasma generated from the liquid medium M.

Specifically, the method of processing the polymer compound P or the method of manufacturing a decomposition product may include the following steps. Hereinafter, the processing of the polymer compound P will be described sequentially for respective steps.

(Mixing Step)

First, before the plasma processing is performed on the liquid medium M, the liquid medium M and the polymer compound P may be mixed in the container 10. Prior to this step, the polymer compound P may be ground. For example, the polymer compound P in a particulate state may be dispersed in the liquid medium M or dissolved in the liquid medium M. In addition, the timing of inputting the polymer compound P into the liquid medium M is not limited to the above-mentioned example. For example, the polymer compound P may be inputted into the liquid medium M at the same time as the plasma processing of the liquid medium M. In addition, after the plasma processing is performed on the liquid medium M, the polymer compound P may be inputted into the liquid medium M on which the plasma processing has been performed.

(Plasma Processing Step)

Next, the plasma processing is performed on the liquid medium M in the container 10. Specifically, as illustrated in FIGS. 1 and 2, in the state in which the liquid medium M and the polymer compound P are accommodated in the container 10, the electromagnetic wave transmission part 22 operates and emits electromagnetic waves to the electromagnetic wave receiving part 24 in the container 10. The core 40 of the electromagnetic wave receiving part 24 receives energy of the electromagnetic waves and emits free electrons from the surface thereof. At least some of the free electrons emitted from the core 40 pass through the dielectric sheath 42 and collide with molecules of the liquid medium M at the periphery of the electromagnetic wave receiving part 24. The collision of the electrons generates plasma from a part of the liquid medium M, thereby emitting light. The excited electrons are caught between the molecules of the liquid medium M. The molecules of the liquid medium M are radicalized and/or ionized. For example, it is estimated that in case that the liquid medium M is ethylene glycol, the attack from the electrons generates charges on carbon atoms, oxygen atoms, and hydrogen atoms in the structure of ethylene glycol. However, the structure of the plasma generated from the liquid medium M is not limited to the flowing description, and various variations, such as a structure having a plurality of charges, may be considered.

[Chemical Formula 1]

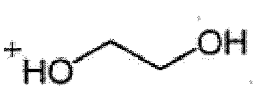
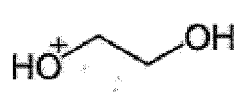
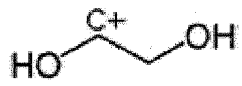
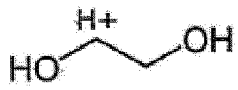
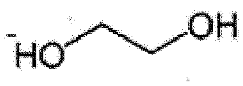
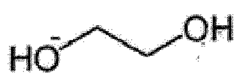
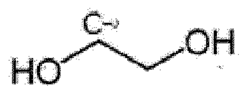
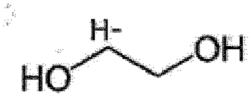

In addition, when the electrons, which do not pass through the dielectric sheath 42 among the electrons emitted from the core 40, are accumulated inside the dielectric sheath 42, dielectric polarization may occur at two opposite sides of the dielectric sheath 42. Therefore, the charges occurring on the outer surface of the dielectric sheath 42 may also excite the liquid medium M.

When the electromagnetic wave transmission part 22 emits electromagnetic waves to the electromagnetic wave receiving part 24 in the state in which the liquid medium M is in contact with the electromagnetic wave receiving part 24 as described above, the core 40 of the electromagnetic wave receiving part 24 may emit free electrons. The emitted electrons pass through the dielectric sheath 42 and/or induce the dielectric polarization of the dielectric sheath

42 and excite the liquid medium M into a plasma state. It is possible to generate radicals of the liquid medium M by performing the plasma processing on the liquid medium M as described above.

(Temperature Condition)

For example, a temperature at which the plasma processing is performed on the liquid medium M (e.g., a temperature of the liquid medium M at which the electromagnetic wave transmission part 22 generates electromagnetic waves) may be, but not particularly limited to, 10°C or more and 150°C or less. For example, a temperature at which the polymer compound P comes into contact with the plasma generated from the liquid medium M may be, but not particularly limited to, 10°C or more and 150°C or less. These temperatures are substantially equal to each other when the plasma processing is performed in the state in which the polymer compound P and the liquid medium M are in contact with each other. In addition, the "temperature" in this case means an average temperature of the entire liquid medium M in the container 10.

For example, the temperature at which the plasma processing is performed may be 15°C or more, 20°C or more, 25°C or more, 30°C or more, 40°C or more, 50°C or more, 60°C or more, 70°C or more, 80°C or more, 90°C or more, or 100°C or more. For example, the temperature at which the plasma processing is performed may be 140°C or less, 120°C or less, 100°C or less, 80°C or less, 60°C or less, 50°C or less, 40°C or less, or 30°C or less.

For example, the temperature at which the polymer compound P comes into contact with the plasma generated from the liquid medium M may be 15°C or more, 20°C or more, 25°C or more, 30°C or more, 40°C or more, 50°C or more, 60°C or more, 70°C or more, 80°C or more, 90°C or more, or 100°C or more. For example, the temperature at which the polymer compound P comes into contact with the plasma generated from the liquid medium M may be 140°C or less, 120°C or less, 100°C or less, 80°C or less, 60°C or less, 50°C or less, 40°C or less, or 30°C or less.

In principle, because an active heating operation need not be performed to perform the plasma processing on the liquid medium M, it is advantageous, in terms of costs and efficiency, to perform the plasma processing on the liquid medium M without heating the container 10 or the liquid medium M. The temperature at which the plasma processing is performed on the liquid medium M and/or the temperature at which the polymer compound P comes into contact with the plasma generated from the liquid medium M may be a room temperature. However, there is a likelihood that the liquid medium M may be heated by molecules of plasma generated from another liquid medium M or the electromagnetic waves (e.g., microwaves). In this case, the temperature of the liquid medium M may increase while the electromagnetic waves are emitted during the plasma processing on the liquid medium M. To prevent evaporation of the liquid medium M, the output of the electromagnetic waves may be adjusted to prevent the temperature from exceeding the boiling point of the liquid medium M.

(PRESSURE CONDITION)

A pressure at which the plasma processing is performed on the liquid medium M and/or a pressure at which the polymer compound P comes into contact with the plasma generated from the liquid medium M may be, but not particularly limited to, 0.05 MPa or more and less than 1.5 MPa. For example, a pressure at which the plasma processing is performed may be 0.06 MPa or more, 0.07 MPa or more, 0.08 MPa or more, 0.09 MPa or more, 0.1 MPa or more, or atmospheric pressure or higher. For example, the pressure at which the plasma processing is performed may be 1.4 MPa or less, 1.3 MPa or less, 1.2 MPa or less, 1.1 MPa or less, or atmospheric pressure or lower.

In principle, because an active pressurization operation need not be performed to perform the plasma processing on the liquid medium M, it is advantageous, in terms of costs and efficiency, to perform the plasma processing on the liquid medium M without pressurizing the internal space of the container 10 or the liquid medium M. Particularly, the plasma processing on the liquid medium M and/or the contact between the polymer compound P and the plasma generated from the liquid medium M may be performed under atmospheric pressure. For example, in case that the container 10 is opened to the outside, the method may be performed under atmospheric pressure.

(Decomposition Step)

The plasma generated from the liquid medium M is in a chemically unstable state and thus has very high reactivity. Therefore, the plasma generated from the liquid medium M reacts with the polymer compound P and decomposes the polymer compound P. For example, unpaired electrons of the radical generated from the liquid medium M attack the polymer compound P. Therefore, one or more bonded structures, among the structures of the polymer compound P, are cut, such that the polymer compound P is decomposed into polymers or oligomers having smaller molecular weights and/or monomers that are minimum constituent units of the polymer compound P. As described above, in a state in which the liquid medium M, which is not converted into plasma, is in contact with the polymer compound P, the plasma processing is performed on the liquid medium M, such that the plasma generated from the liquid medium M may

decompose the polymer compound P. More specifically, in a state in which the polymer compound P is dissolved or dispersed in the liquid medium M that is not converted into plasma, the plasma processing is performed on the liquid medium M, such that the plasma generated from the liquid medium M may decompose the polymer compound P. As the polymer compound P comes into contact with the plasma generated from the liquid medium M as described above, the decomposition product decomposed from the polymer compound P is produced.

The decomposition of the polymer compound P does not require a catalyst. That is, the polymer compound P comes into contact with the plasma generated from the liquid medium M in a state in which no catalyst for decomposition reaction for the polymer compound is present. Therefore, this process is efficient because costs may be reduced in comparison with the decomposition processing using a catalyst.

(Recovery Step)

The generated decomposition product is separated and recovered from the liquid medium M and the remaining polymer compound P. The method of recovering the generated decomposition product is not particularly limited and may be performed by using one or more existing techniques. Examples of the recovery method may include drying, filtration, extraction, distillation, purification, and chromatography. Examples of chromatography may include high-performance liquid chromatography (HPLC) and ultra-performance liquid chromatography (UPLC). For example, in case that the decomposition product is dissolved in the liquid medium M and the polymer compound P is not dissolved in the liquid medium M, the polymer compound P and the liquid medium M in which the decomposition product is dissolved are separated first. Thereafter, the decomposition product may be recovered by separating the decomposition product from the liquid medium M. In this way, with the processing of the polymer compound P, the decomposition product may be manufactured from the polymer compound P.

According to the processing apparatus 1 and the method described above, the decomposition of the polymer compound P may be efficiently performed without requiring a catalyst and performing an active heating or pressurization operation. Therefore, it is possible to efficiently reuse the polymer compound P. Therefore, it is possible to contribute to a reduction in environment load or an implementation of circular economy.

<Second Embodiment>

A processing apparatus 1 according to a second embodiment and a processing method using the processing apparatus 1 will be described with reference to FIG. 3.

The second embodiment differs from the first embodiment in that the plasma generator 20 is positioned outside the container 10. Hereinafter, a difference between the embodiments will be mainly described, and a repeated description of the common parts of the embodiments will not be described.

FIG. 3 is a cross-sectional view illustrating the processing apparatus 1 according to the second embodiment. As illustrated in FIG. 3, the electromagnetic wave transmission part 22 and the electromagnetic wave emission part 32 are positioned outside the container 10. The electromagnetic waves emitted from the electromagnetic wave transmission part 22 pass through the sidewall 14 of the container 10 and reach the electromagnetic wave receiving part 24 in the container 10.

As described above, the sidewall 14 of the container 10 needs to transmit the electromagnetic wave (e.g., microwave). Therefore, unlike the first embodiment, at least the electromagnetic wave transmission part 22 in the container 10 is made of nonmetallic material. Examples of the material of the sidewall 14 may include glass, ceramics, rubber, and plastic.

In the processing apparatus 1 according to the second embodiment, the electromagnetic wave transmission part 22 is positioned outside the container 10, such that the degradation of the electromagnetic wave transmission part 22 caused by the contact with the liquid medium M may be prevented. Meanwhile, because the processing apparatus 1 according to the first embodiment disposed in the container 10 and emits the electromagnetic waves, the processing apparatus 1 is advantageous because the electromagnetic waves are not attenuated by the container 10.

<Modified Example>

The shape or size of the electromagnetic wave receiving part 24 is not limited to the above-mentioned example. For example, the electromagnetic wave receiving part 24 may have any shape such as a chamber shape, a film shape, a plate shape, a stand shape, a column shape, a net shape, a fiber shape, and a spherical shape. The electromagnetic wave receiving part 24 may be in contact with the bottom wall 12 or the sidewall 14 of the container 10, supported at a position spaced apart from the container 10, or floated in the liquid medium M.

For example, the electromagnetic wave receiving part 24 may have a film or plate shape having a large area. The electromagnetic wave receiving part 24 may have a shape such as a stand on the bottom wall 12 and thus serve

as a table for mounting the polymer compound P that is a processing object. The electromagnetic wave receiving part 24 may have a small spherical shape having a core-shell structure including the core 40 and the dielectric sheath 42. The shape of the electromagnetic wave receiving part 24 may have a plurality of concave-convex portions. This shape having the concave-convex portions is advantageous because the discharge is easily generated from the electromagnetic wave receiving part 24.

[Mode for Invention]

<Example 1>

First, the processing apparatus 1 illustrated in FIG. 1 was prepared. A microwave output device (model name: GMP30K) manufactured by Sairem was used as the electromagnetic wave transmission part 22. A copper wire coated with $SiO_2$ glass was used as the electromagnetic wave receiving part 24. Ethylene glycol (EG), as the liquid medium M, was inputted into the container 10 made of $SiO_2$ glass. Next, powder made by grinding polyethylene terephthalate (PET) into small pieces was prepared as the polymer compound P of the processing object, and the powder was inputted into the container 10 and dispersed in ethylene glycol. The amount of inputted PET and ET was set so that a mass ratio between PET and ET was 3:97.

Next, the electromagnetic wave output part 30 was operated, and the electromagnetic wave emission part 32 emitted microwaves to the electromagnetic wave receiving part 24 in the atmosphere. The output of the microwave was set to 300 W, and the emission of the microwaves was continuously performed for 10 minutes. The ethylene glycol was heated by the microwaves, such that the temperature of the ethylene glycol was maintained to be about 100°C while the emission was performed continuously. As a result, a spark was generated at the periphery of the electromagnetic wave receiving part 24, and then plasma light-emitting was continuously observed on the electromagnetic wave receiving part 24.

After the microwaves was emitted for 10 minutes, the decomposition product dissolved in ethylene glycol in the container 10 was detected by using an ultra-performance liquid chromatography device (model name: ACQUITY UPLC system D) manufactured by Water. The analysis conditions were set as follows.

- Stationary phase: ACQUITY UPLC BEH C8 1.7 um 100 mm
- Mobile phase: methanol/water=7/3 (v/v)
- Method of preparing measuring solution: the measuring solution was manufactured by diluting the decomposition product with methanol/water=7/3(v/v) and then filtering the decomposition product with a syringe filter.
- Flow velocity: 0.4 mL/min
- Detection wavelength: 254 nm

As a result, it was detected that the monomer, dimer, and trimer of the bis-2-hydroxyethyl terephthalate (BHET) were dissolved in ethylene glycol. The monomer, dimer, and trimer of the detected BHET were considered as the decomposition products, and the undetected components were considered as non-decomposition products.

The amount of detected decomposition product was converted into the number of moles of PET, which was a raw material, and a value made by dividing the converted number of moles of PET by the overall number of moles of PET inputted as a raw material was calculated as a "decomposition ratio". That is, the "decomposition ratio" refers to a ratio of a value, which is made by converting the amount of monomer, dimer, and trimer of the detected BHET into the amount of substance of PET, to the amount of substance of inputted PET. The decomposition ratio was 37%. That is, it was ascertained that 37 mol% of inputted PET were decomposed into the monomer, dimer, or trimer of the BHET by the processing apparatus 1.

<Example 2>

PET was processed in the same way as Example 1, except that the microwave emission time was increased from 10 minutes to 25 minutes. The decomposition ratio of PET was 87%.

<Comparative Example 1>

PET was processed in the same way as Example 1, except that an oil bath was used to maintain a temperature of the container 10 at 100°C without emitting microwaves and installing the electromagnetic wave receiving part 24 in the container 10. The decomposition of PET was not observed, and the decomposition ratio was 0%.

<Comparative Example 2>

PET was processed in the same way as Example 1, except that a microwave output was adjusted while microwaves were emitted to maintain a temperature of ethylene glycol, which was a liquid medium, at about 100°C without installing the electromagnetic wave receiving part 24 in the container 10. The decomposition of PET was not observed, and the decomposition ratio was 0%.

The experimental conditions of Examples and Comparative Examples and the decomposition ratio of PET are summarized in Table 1 below.

[Table 1]

| | PET | EG | Electroma gnetic wave Receiving part | Electrom agnetic wave Output | Cata lyst | Temper ature Condit ion | Pressure Condition | Emission Time | Decompo sition ratio |
|---|---|---|---|---|---|---|---|---|---|
| Example 1 | 3 wt% | 97 wt% | Presence | 300 W | Abse nce | 100°C | Atmospheric pressure | 10 minutes | 37% |
| Example 2 | 3 wt% | 97 wt% | Presence | 300 W | Abse nce | 100 °C | Atmospheric pressure | 25 minutes | 87% |
| Comparat ive Example 1 | 3 wt% | 97 wt% | Absence | - | Abse nce | 100 °C | Atmospheric pressure | - | 0% |
| Comparat ive Example 2 | 3 wt% | 97 wt% | Absence | 300 W | Abse nce | 100 °C | Atmospheric pressure | - | 0% |

## Claims

1. A processing apparatus for a polymer compound, the processing apparatus comprising:

   a container configured to accommodate a polymer compound and a liquid medium; and
   a plasma generator disposed in the container and configured to perform plasma processing on the liquid medium.

2. The processing apparatus of claim 1, wherein the plasma generator, which performs the plasma processing on the liquid medium in the container, comprises:

   an electromagnetic wave transmission part configured to transmit electromagnetic waves; and
   an electromagnetic wave receiving part configured to receive the electromagnetic waves from the electromagnetic wave transmission part.

3. The processing apparatus of claim 2, wherein the electromagnetic wave receiving part performs the plasma processing on the liquid medium in response to the reception of the electromagnetic waves.

4. The processing apparatus of claim 2, wherein the electromagnetic wave is a microwave with a frequency of 100 MHz or more and 300 GHz or less.

5. The processing apparatus of claim 2, wherein the electromagnetic wave receiving part includes a metallic material.

6. The processing apparatus of claim 2, wherein the electromagnetic wave receiving part comprises:

   a core having conductivity; and
   a dielectric material sheath configured to at least partially cover the core.

7. The processing apparatus of claim 2, wherein the electromagnetic wave receiving part is disposed inside the container and comes into contact with the liquid medium.

8. The processing apparatus of claim 2, wherein at least a part of the electromagnetic wave transmission part is disposed inside the container.

9. The processing apparatus of claim 2, wherein the electromagnetic wave transmission part is disposed outside the container, and a portion of the container, through which the electromagnetic waves from the electromagnetic wave transmission part at least pass, is made of a nonmetallic material.

10. A method, which manufactures a decomposition product by processing a polymer compound, the method comprising: bringing the polymer compound into contact with plasma generated from a liquid medium.

11. The method of claim 10, further comprising:
    emitting electromagnetic waves to the electromagnetic wave receiving part in a state in which the liquid medium is in contact with the electromagnetic wave receiving part.

12. The method of claim 11, wherein the electromagnetic wave receiving part performs the plasma processing on the liquid medium in response to the reception of the electromagnetic waves.

13. The method of claim 10, further comprising:
    generating a radical of the liquid medium by performing plasma processing on the liquid medium.

14. The method of claim 10, further comprising:
    performing plasma processing on the liquid medium in a state in which the polymer compound is dissolved or dispersed in the liquid medium that is not converted into plasma.

15. The method of claim 10, wherein the polymer compound comes into contact with the plasma generated from the liquid medium under a condition of a temperature of 10°C or more and 150°C or less.

16. The method of claim 10, wherein the polymer compound configured to come into contact with the plasma generated

from the liquid medium under a condition of a pressure of 0.05 MPa or more and less than 1.5 MPa.

**17.** The method of claim 10, wherein the liquid medium includes polar molecules.

**18.** The method of claim 10, wherein the polymer compound comes into contact with the plasma generated from the liquid medium in a state in which no catalyst for a decomposition reaction for a polymer compound is present.

**19.** The method of claim 10, wherein a decomposition product decomposed from the polymer compound is produced as the polymer compound comes into contact with the plasma generated from the liquid medium.

**20.** A method, which processes a polymer compound, the method comprising:
bringing the polymer compound into contact with plasma generated from a liquid medium.

[Figure 1]

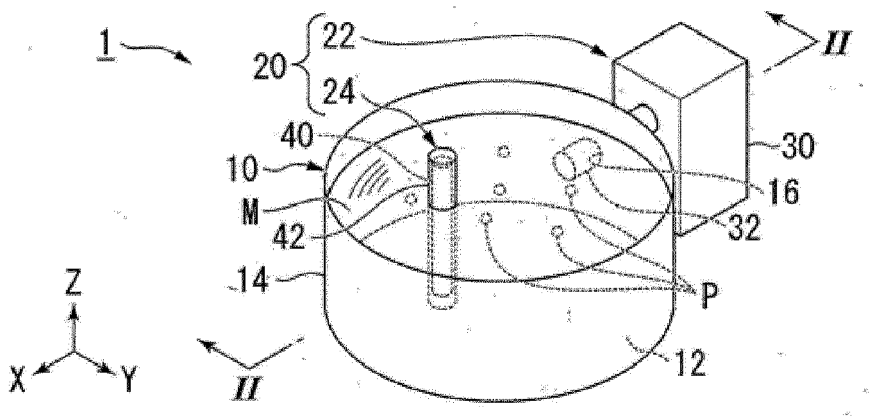

[Figure 2]

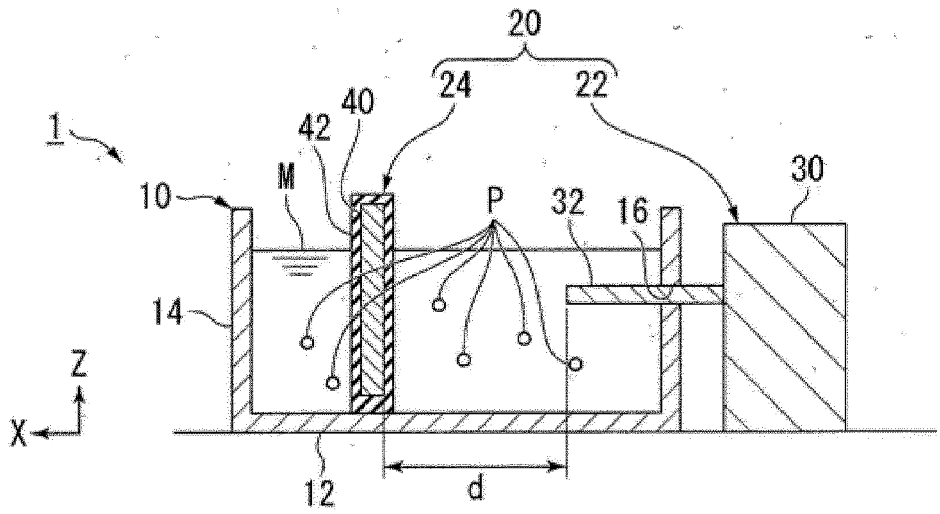

[Figure 3]

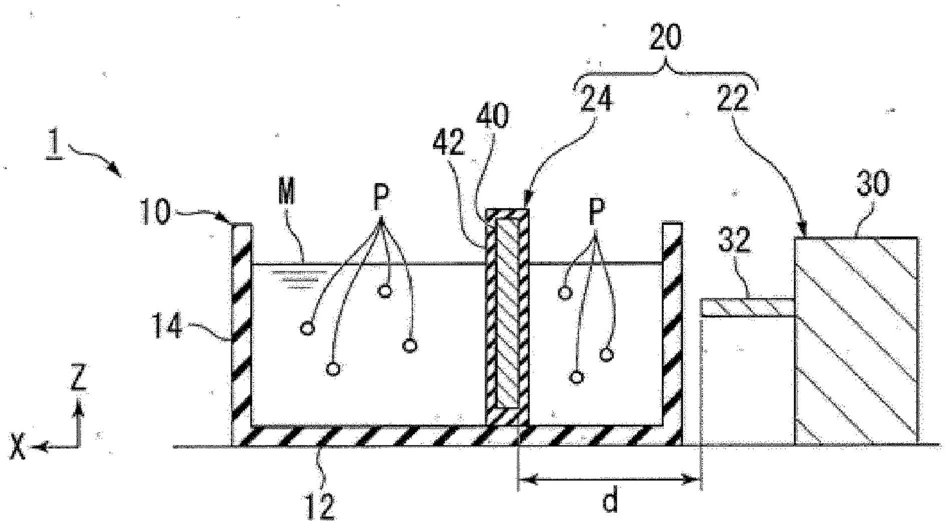

## INTERNATIONAL SEARCH REPORT

International application No.
**PCT/KR2022/014120**

**A. CLASSIFICATION OF SUBJECT MATTER**

**C08J 11/10**(2006.01)i; **C08J 11/14**(2006.01)i; **C08J 11/22**(2006.01)i

According to International Patent Classification (IPC) or to both national classification and IPC

**B. FIELDS SEARCHED**

Minimum documentation searched (classification system followed by classification symbols)

C08J 11/10(2006.01); A01M 1/02(2006.01); A01M 1/22(2006.01); A62D 3/00(2007.01); B01J 19/08(2006.01); C08J 3/28(2006.01); C08J 5/18(2006.01); C11D 11/00(2006.01); H01L 21/02(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 고분자(polymer), 액체(liquid), 분해 생성물(decomposition products), 플라즈마 (plasma)

**C. DOCUMENTS CONSIDERED TO BE RELEVANT**

| Category* | Citation of document, with indication, where appropriate, of the relevant passages | Relevant to claim No. |
|---|---|---|
| Y | KR 10-2007-0113313 A (MITSUBISHI RAYON CO., LTD. et al.) 28 November 2007 (2007-11-28)<br>See claim 1; paragraphs [0027]-[0067]; and figure 1. | 1-20 |
| Y | 김영국 등. BHET와 플라즈마 상호작용을 통한 합성가스 CO와 $H_2$의 생성. 한국생산제조학회 학술발표대회 논문집. 2020, p. 252 (KIM, Young Kuk et al. CO and $H_2$ Syngas production from BHET-plasma interaction. Proceedings of KSMTE Annual Autumn Conference.)<br>See page 252. | 1-20 |
| A | KR 10-2020-0144653 A (UCF-HYU (INDUSTRY-UNIVERSITY COOPERATION FOUNDATION HANYANG UNIVERSITY)) 30 December 2020 (2020-12-30)<br>See entire document. | 1-20 |
| A | KR 10-2009-0108874 A (KOREA BASIC SCIENCE INSTITUTE) 19 October 2009 (2009-10-19)<br>See entire document. | 1-20 |
| A | KR 10-2019-0087323 A (TOKYO ELECTRON LIMITED) 24 July 2019 (2019-07-24)<br>See entire document. | 1-20 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 January 2023** | **17 January 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2022/014120**

| Patent document cited in search report | Publication date (day/month/year) | Patent family member(s) | Publication date (day/month/year) |
|---|---|---|---|
| KR 10-2007-0113313 A | 28 November 2007 | JP 2008-104043 A1 | 04 September 2008 |
| | | JP 2009-104043 A5 | 07 May 2009 |
| | | JP 2013-031842 A | 14 February 2013 |
| | | JP 5518281 B2 | 11 June 2014 |
| | | JP 5725304 B2 | 27 May 2015 |
| | | KR 10-0938323 B1 | 22 January 2010 |
| | | TW 200637648 A | 01 November 2006 |
| | | TW I405608 B | 21 August 2013 |
| | | US 2008-0210664 A1 | 04 September 2008 |
| | | WO 2006-104043 A1 | 05 October 2006 |
| KR 10-2020-0144653 A | 30 December 2020 | KR 10-2266926 B1 | 18 June 2021 |
| KR 10-2009-0108874 A | 19 October 2009 | None | |
| KR 10-2019-0087323 A | 24 July 2019 | CN 110047726 A | 23 July 2019 |
| | | CN 110047726 B | 04 May 2021 |
| | | JP 2019-125685 A | 25 July 2019 |
| | | JP 6799549 B2 | 16 December 2020 |
| | | TW 201933478 A | 16 August 2019 |
| | | US 10734204 B2 | 04 August 2020 |
| | | US 2019-0221406 A1 | 18 July 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022062848 A
- JP 2008088096 A
- JP 2015168741 A